# EUROPEAN PATENT SPECIFICATION

(11) **EP 2 069 323 B1**
(45) Date of publication and mention of the grant of the patent: **05.05.2010**
(21) Application number: 07836498.1
(22) Date of filing: 03.08.2007
(51) Int. Cl.: C07D 333/34, C07D 413/12, C07D 513/04

(54) **PROCESSES FOR THE PREPARATION OF N-(2-ACETYL-4,6-DIMETHYLPHENYL)-3-{[(3,4 DIMETHYL-5-ISOXAZOLYL)AMINO]SULFONYL}-2-THIOPHENECARBOXAMIDE**
VERFAHREN ZUR HERSTELLUNG VON N-(2-ACETYL-4,6-DIMETHYLPHENYL)-3-{[(3,4 DIMETHYL-5-ISOXAZOLYL)AMINO]SULFONYL}-2-THIOPHENCARBOXAMID
PROCÉDÉS DE PRÉPARATION DE N-(2-ACÉTYL-4,6-DIMÉTHYLPHÉNYL)-3-{[(3,4 DIMÉTHYL-5-ISOXAZOLYL)AMINO]SULFONYL}-2- THIOPHÈNECARBOXAMIDE

(30) Priority: 04.08.2006 US 835636 P
(43) Date of publication of application: 17.06.2009
(73) Proprietor: ENCYSIVE PHARMACEUTICALS, INC., New York, NY 10017-5755 (US)
(72) Inventor: REICHWEIN, John F., Houston, TX 77081 (US)
(74) Representative: Laurent, Claire
(86) International application number: PCT/US2007/017391
(87) International publication number: WO 2008/019096

(56) References cited:
- US-A1- 2001 056 183

## Description

### FIELD

Provided herein are processes for the preparation of N-(2-acetyl-4,6-dimethylphenyl)-3-{[(3,4 dimethyl-5-isoxazolyl)amino]sulfonyl}-2-thiophenecarboxamide, a compound useful for the treatment of endothelin-mediated disorders.

### BACKGROUND

N-(2-acetyl-4,6-dimethylphenyl)-3-{[(3,4 dimethyl-5-isoxazolyl)amino]sulfonyl}-2-thiophenecarboxamide is an endothelin antagonist (U.S. Pat. No. 6,686,382). Endothelin antagonists are useful for the treatment of hypertension such as peripheral circulatory failure, heart disease such as angina pectoris, cardiomyopathy, arteriosclerosis, myocardial infarction, pulmonary hypertension, vasospasm, vascular restenosis, Raynaud's disease, cerebral stroke such as cerebral arterial spasm, cerebral ischemia, late phase cerebral spasm after subarachnoid hemorrhage, asthma, bronchoconstriction, renal failure, particularly post-ischemic renal failure, cyclosporine nephrotoxicity such as acute renal failure, colitis, as well as other inflammatory diseases, endotoxic shock caused by or associated with endothelin, and other diseases in which endothelin has been implicated. Provided herein are processes for the preparation of N-(2-acetyl-4,6-dimethylphenyl)-3-{[(3,4 dimethyl-5-isoxazolyl)amino]sulfonyl}-2-thiophenecarboxamide.

### SUMMARY

Provided herein are processes for the preparation of N-(2-acetyl-4,6-dimethylphenyl)-3-{[(3,4 dimethyl-5-isoxazolyl)amino]sulfonyl}-2-thiophenecarboxamide: 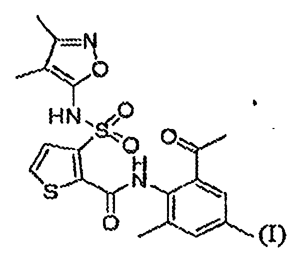 or a pharmaceutically acceptable salt, enol ether, enol ester, ketal, hemiketal, solvate or hydrate thereof.

In one aspect, provided is a process for preparing the compound of Formule (I), or a pharmaceutically acceptable salt, enol ether, enol ester, ketal, hemiketal, solvate or hydrate thereof, wherein the process involves a step of reacting a compound of Formula (II): 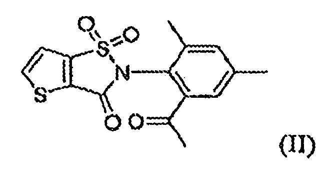 with a compound of Formula (III): 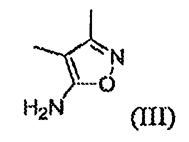 or a salt thereof.

### DETAILED DESCRIPTION

### Definitions

Unless defined otherwise, all technical and scientific therms used herein have the same meaning as is commonly understood by one of ordinary skill in the art to which this invention belongs. In the event that there are a plurality of definitions for a term herein, those in this section prevail unless stated otherwise.

Pharmaceutically acceptable derivatives of a compound such as salts, enol ethers, enol esters, ketals, orthoesters, hemiketals, solvates, and hydrates thereof may be readily prepared by those of skill in this art using known methods for such derivatization. The compounds produced may be administered to animals or humans without substantial toxic effects and either are pharmaceutically active or are prodrugs. Pharmaceutically acceptable salts include, but are not limited to, amine salts, such as but not limited to N,N'-dibenzylethylenediamine, chloroprocaine, choline, ammonia, diethanolamine and other hydroxyalkylamines, ethylenediamine, N-methylglucamine, procaine, N-benzylphenethylamine, 1-para-chlorobenzyl-2-pyrrolidin-1'-ylmethyl-benzimidazole, diethylamine and other alkylamines, piperazine and tris(hydroxymethyl)aminomethane; alkali metal salts, such as but not limited to lithium, potassium and sodium; alkali earth metal salts, such as but not limited to barium, calcium and magnesium; transition metal salts, such as but not limited to zinc; and other metal salts, such as but not limited to sodium hydrogen phosphate and disodium phosphate; and also including, but not limited to, salts of mineral acids, such as but not limited to hydrochlorides and sulfates; and salts of organic acids, such as but not limited to acetates, lactates, malates, tartrates, citrates, ascorbates, succinates, butyrates, valerates and fumarates. Pharmaceutically acceptable esters include, but are not limited to, alkyl, alkenyl, alkynyl, alk(en)(yn)yl, aryl, aralkyl, and cycloalkyl esters of carboxylic acids. Pharmaceutically acceptable enol ethers include, but are not limited to, derivatives of formula C=C(OR) where R is hydrogen, alkyl, alkenyl, alkynyl, alk(en)(yn)yl, aryl, aralkyl, or cycloalkyl, Pharmaceutically acceptable enol esters include, but are not limited to, derivatives of formula C=C(OC(O)R) where R is hydrogen, alkyl, alkenyl, alkynyl, aryl, aralkyl, or cycloalkyl. Pharmaceutically acceptable solvates and hydrates are complexes of a compound with one or more solvent or water molecules, or 1 to about 100, or 1 to about 10, or one to about 2, 3 or 4, solvent or water molecules.

It is to be understood that the compounds provided herein may contain chiral centers. Such chiral centers may be of either the (R) or (S) configuration, or may be a mixture thereof. Thus, the compounds provided herein may be enantiomerically pure, or be stereoisomeric or diastereomeric mixtures. It is to be understood that the chiral centers of the compounds provided herein may undergo epimerization *in vivo.* As such, one of skill in the art will recognize that administration of a compound in its (R) form is equivalent, for compounds that undergo epimerization *in vivo,* to administration of the compound in its (S) form.

As used herein, alkyl refers to an unbranched or branched hydrocarbon chain. An alkyl group may be unsubstituted or substituted with one or more heteroatoms.

As used herein, alkenyl refers to an unbranched or branched hydrocarbon chain comprising one or more double bonds. The double bond of an alkenyl group may be unconjugated or conjugated to another unsaturated group. An alkenyl group may be unsubstituted or substituted with one or more heteroatoms.

As used herein, alkynyl refers to an unbranched or branched hydrocarbon chain comprising one of more triple bonds therein. The triple bond of an alkynyl group may be unconjugated or conjugated to another unsaturated group. An alkynyl group may be unsubstituted or substituted with one or more heteroatoms.

As used herein, alk(en)(yn)yl refers to an unbranched or branched hydrocarbon group comprising at least one double bond and at least one triple bond. The double bond or triple bond of an alk(en)(yn)yl group may be unconjugated or conjugated to another unsaturated group. An alk(en)(yn)yl group may be unsubstituted or substituted with one or more heteroatoms.

Exemplary alkyl, alkenyl, alkynyl, and alk(en)(yn)yl groups herein include, but are not limited to, methyl, ethyl, propyl, isopropyl, isobutyl, n-butyl, sec-butyl, tert-butyl, isopentyl, neopentyl, tert-pentyl, isohexyl, allyl (propenyl) and propargyl (propynyl).

As used herein, "aryl" refers to aromatic monocyclic or multicyclic groups containing from 6 to 19 carbon atoms. Aryl groups include, but are not limited to groups such as unsubstituted or substituted fluorenyl, unsubstituted or substituted phenyl, and unsubstituted or substituted naphthyl.

As used herein, "heteroaryl" refers to a monocyclic or multicyclic aromatic ring system, in certain embodiments, of about 5 to about 15 members where one or more, in one embodiment 1 to 3, of the atoms in the ring system is a heteroatom, that is, an element other than carbon, including but not limited to, nitrogen, oxygen or sulfur. The heteroaryl group may be optionally fused to a benzene ring. Heteroaryl groups include, but are not limited to, furyl, imidazolyl, pyrimidinyl, tetrazolyl, thienyl, pyridyl, pyrrolyl, thiazolyl, isothiazolyl, oxazolyl, isoxazolyl, triazolyl, quinolinyl or isoquinolinyl.

As used herein, "halo," "halogen," or "halide" refers to F, Cl, Br or I.

As used herein, base refers to any compound that accepts protons in water or solvent. Thus, exemplary bases include, but are not limited to, alkali metal hydroxides and alkali metal alkoxides (i.e., MOR, wherein M is an alkali metal such as but not limited to potassium, lithium, or sodium and R is hydrogen, alkyl, alkenyl, alkynyl, or alk(en)(yn)yl) such as but not limited to potassium hydroxide, potassium tert-butoxide, potassium tert-pentoxide, sodium hydroxide, sodium tert-butoxide, lithium hydroxide, etc.); other hydroxides such as but not limited to magnesium hydroxide (Mg(OH)₂), calcium hydroxide (Ca(OH)₂), or barium hydroxide (Ba(OH)₂); alkali metal hydrides (i.e., MH, wherein M is as defined above) such as but not limited to sodium hydride, potassium hydride, or lithium hydride; carbonates such as but not limited to potassium carbonate (K₂CO₃), sodium carbonate (Na₂CO₃), potassium bicarbonate (KHCO₃), or sodium bicarbonate (NaHCO₃); alkyl ammonium hydroxides, alkenyl ammonium hydroxides, alkynyl ammonium hydroxides, or alk(en)(yn)yl ammonium hydroxides such as but not limited to n-tetrabutyl ammonium hydroxide (TBAH); amines such as ammonia, diethylamine, 2,2,6,6-tetramethyl piperidine (HTMP), tertiary amines (such as but not limited to dimethylethyl amine, diisopropylethylamine, trimethylamine, triethylamine, tributylamine, N-methylmorpholine, N-methyl pyrrolidine, 1,8-diazabicyclo[5.4.0]-7-undecene (DBU), 1,5-diazabicylo[4.3.0]-5-nonene (DBN), or tetramethylenediamine (TMEDA)), aromatic amines (such as but not limited to pyridine, collidine, lutidine, picoline, quinoline, or N,N-dimethylaniline); alkali metal amides such as but not limited to lithium amide, lithium dimethylamide, lithium diisopropylamide (LDA), lithium tetramethylpiperidide (LiTMP), or alkali metal hexamethyldisilazanes (such as but not limited to potassium hexamethyldisilazane, (KHMDS), sodium hexamethyldisilazane (NaHMDS), or lithium hexamethyldisilazane (LiHMDS)); alkyl lithiums, alkenyl lithiums, alkynyl lithiums, or alk(en)(yn)yl lithiums such as but not limited to n-butyl lithium sec-butyllithium, isopropyllithium; alkyl magnesium halides, alkenyl magnesium halides, alkynyl magnesium halides, or alk(en)(yn)yl magnesium halides such as but not limited to methyl magnesium bromide.

As used herein, solvent refers to any liquid that completely or partially dissolves a solid, liquid, or gaseous solute, resulting in a solution such as but not limited to hexane, benzene, toluene, diethyl ether, chloroform, ethyl acetate, dichloromethane, carbon tetrachloride, 1,4-dioxane, tetrahydrofuran, glyme, diglyme, acetone, acetonitrile, dimethylformamide, dimethyl sulfoxide, dimethylacetamide, or N-methyl-2-pyrrolidone.

As used herein, dehydrating agent refers to any compound that promotes the formation of carboxamides from carboxylic acids, such as but not limited to thionyl chloride, sulfuryl chloride, a carbodiimide, an anhydride or a mixed anhydride, a phenol (such as but not limited to nitrophenol, pentafluorophenol, or phenol), or a compound of Formula (A): 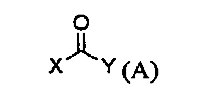 wherein each of X and Y is independently an unsubstituted or substituted heteroaryl group (such as but not limited to imidazolyl, benzimidazolyl, or benzotriazolyl). Examples of dehydrating agents further include, but are not limited to, benzotriazole-1-yl-oxy-tris-(dimethylamino)-phosphonium hexafluorophosphate (BOP), N,N'-carbonyldiimidazole (CDI), 3-(diethoxyphosphoryloxy)-1,2,3-benzotriazin-4(3H)-one (DEPBT), 1-ethyl-3-(3-dimethyllaminopropyl)carbodiimide (EDC), 2-(7-aza-1H-benzotriazole-1-yl)-1,1,3,3-tetramethyluronium hexafluorophosphate (HATU), 2-(1H-benzotriazole-1-yl)-1,1,3,3-tetramethyluronium hexafluorophosphate (HBTU), 1-hydroxybenzotriazole (HOBt), benzotriazole-1-yl-oxy-tris-pyrrolidino-phosphonium hexafluorophosphate (PyBOP), 2-(1H-benzotriazole-1-yl)-1,1,3,3-tetramethyluronium tetrafluoroborate (TBTU), O-(3,4-dihydro-4-oxo-1,2,3-benzotriazine-3-yl)-N,N,N,N-tetramethyluronium tetrafluoroborate (TDBTU), 3-(diethyloxyphosphoryloxy)-1,2,3-benzotriazin-4(3H)-one (DEPBT), dicyclohexylcarbodiimide (DCC), N,N'-diisopropylcarbodiimide (DIC), or 1-hydroxy-7-azabenzotriazole (HOAt).

As used herein, acid refers to any compound that contains hydrogen and dissociates in water or solvent to produce positive hydrogen ions, as well as Lewis acids, including but not limited to hydrochloric acid, sulfuric acid, phosphoric acid, acetic acid, trihaloacetic acids (such as but not limited to trifluoroacetic acid or trichloroacetic acid), hydrogen bromide, maleic acid, sulfonic acids (such as but not limited to toluenesulfonic acids or camphorsulfonic acids), propionic acids (such as but not limited to (R)-chloropropionic acid), phthalamic acids (such as but not limited to N-[(R)-1-(1-naphthyl) ethyl]phthalamic acid), tartaric acids (such as but not limited to L-tartaric acid or dibenzyl-L-tartaric acid), lactic acids, camphoric acids, aspartic acids, or citronellic acids.

It is to be understood that reactants, compounds, solvents, acids, bases, catalysts, agents, reactive groups, or the like may be added individually, simultaneously, separately, and in any order. Furthermore, it is to be understood that reactants, compounds, acids, bases, catalysts, agents, reactive groups, or the like may be predissolved in solution and added as a solution (including, but not limited to, aqueous solutions). In addition, it is to be understood that reactants, compounds, solvents, acids, bases, catalysts, agents, reactive groups, or the like may be in any molar ratio.

It is to be understood that reactants, compounds, solvents, acids, bases, catalysts, agents, reactive groups, or the like may be formed in situ.

### Processes

Provided herein is a process for preparing a compound of Formula (I) or a pharmaceutically acceptable derivative thereof involving the step of reacting a compound of Formula (II) with the a compound of Formula (III) or a salt thereof as depicted in Scheme A below.

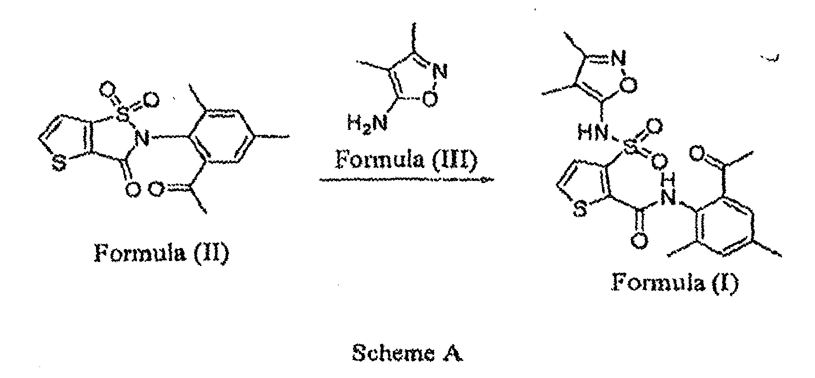

The preparation of the compound of Formula (I) or a pharmaceutically acceptable salt, enol ether, enol ester, ketal hemiketal, solvate or hydrate thereof as depicted in Scheme A may occur in the presence of any base or any combination of bases. In some embodiments, the base is (or combination of bases are) generated in situ, In some embodiments, the base is (or the combination of bases includes) an alkali metal hydride. In some embodiments, the base is sodium hydride.

The preparation of the compound of Formula (I) or a pharmaceutically acceptable salt, enol ether, enol ester, ketal heminetal, solvate or hydrate thereof as depicted in Scheme A may occur in any solvent or any combination of solvents. In some embodiments, the solvent is (or the combination of solvents contains) 1,4-dioxane, tetrahydrofuran, acetone, acetonitrile, dimethylformamide, dimethyl sulfoxide, glyme, diglyme, dimethylacetamide, or N-methyl-2-pyrrolidone. In some embodiments, the solvent is dimethylformamide.

In some embodiments of the preparation of the compound of Formula (I) or a pharmaceutically acceptable salt enol ether, enol ester, ketal hemiketal, solvate or hydrate thereof as depicted in Scheme A, the base is sodium hydride and the solvent is dimethylformamide.

The preparation of the compound of Formula (I) or a pharmaceutically acceptable salt enol ether, enol ester, ketal hemiketal, solvate or hydrate thereof as depicted in Scheme A may occur at any reaction temperature. In some embodiments, the reaction temperature may vary from about - 78°C to about 100°C, In some embodiments, the reaction temperature may vary from about -50°C to about 80°C. In some embodiments, the reaction temperature may vary from about -40C to about 60°C. In some embodiments, the reaction temperature may vary from about -25°C to about 30°C. In some embodiments, the reaction temperature may vary from about -10°C to about 10°C. In some embodiments, the reaction temperature is about 0°C.

In some embodiments of the preparation of the compound of Formula (I) or a pharmaceutically acceptable salt, enol ether, enol ester, ketal, hemiketal, solvate or hydrate thereof as depicted in Scheme A, the base is sodium hydride, the solvent is dimethylformamide, and the reaction temperature is about 0°C.

The preparation of the compound of Formula (I) or a pharmaceutically acceptable salt, enol ether, enol ester, ketal, hemiketal, solvate or hydrate thereof as depicted in Scheme A may occur at any reaction time according to a person of ordinary skill in the art. In some embodiments, the reaction time is from about 30 minutes to about 24 hours. In some embodiments, the reaction time is from about 2 hours to about 3 hours.

In some embodiments of the preparation of the compound of Formula (I) or a pharmaceutically acceptable salt, enol ether, enol ester, ketal, hemiketal, solvate or hydrate thereof as depicted in Scheme A, the base is sodium hydride, the solvent is dimethylformamide, the reaction temperature is about 0°C, and the reaction time is from about 2 hours to about 3 hours.

The preparation of the compound of Formula (I) or a pharmaceutically acceptable salt, enol ether, enol ester, ketal, hemiketal, solvate or hydrate thereof as depicted in Scheme A may occur at any molar ratio according to a person of ordinary skill in the art. In some embodiments, the molar ratio of the compound of Formula (III) to the compound of Formula (II) is from about 3:1 to about 1:1. In other embodiments, the molar ratio of the compound of Formula (II) to the compound of Formula (III) is from about 3:1 to about 1:1. In some embodiments, the molar ratio of the compound of Formula (III) to the compound of Formula (II) is about 1:1.

In one embodiment of the preparation of the compound of Formula (I) or a pharmaceutically acceptable salt, enol ether, enol ester, ketal, hemiketal, solvate or hydrate thereof as depicted in Scheme A, the base is sodium hydride, the solvent is dimethylformamide, the reaction temperature is about 0°C, the reaction time is from about 2 hours to about 3 hours, and the molar ratio is about 1:1.

In another embodiment, provided is a process for preparing a compound of Formula (II) or a pharmaceutically acceptable salt, enol ether, enol ester, ketal, hemiketal, solvate or hydrate thereof by reacting a compound of Formula (IV) with a dehydrating agent or a combination of dehydrating agents as depicted in Scheme B.

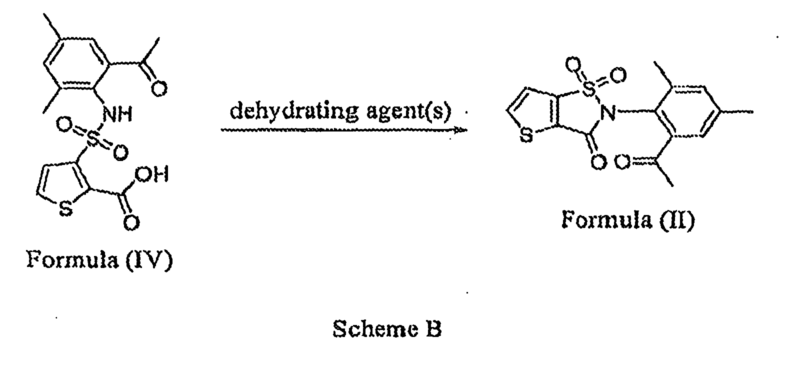

In another embodiment, provided is a process for preparing a compound of Formula (II) or a pharmaceutically acceptable salt, enol ether, enol ester, ketal, hemiketal, solvate or hydrate thereof by reacting a compound of Formula (IVa) with a dehydrating agent of a combination of dehydrating agents as depicted in Scheme B1.

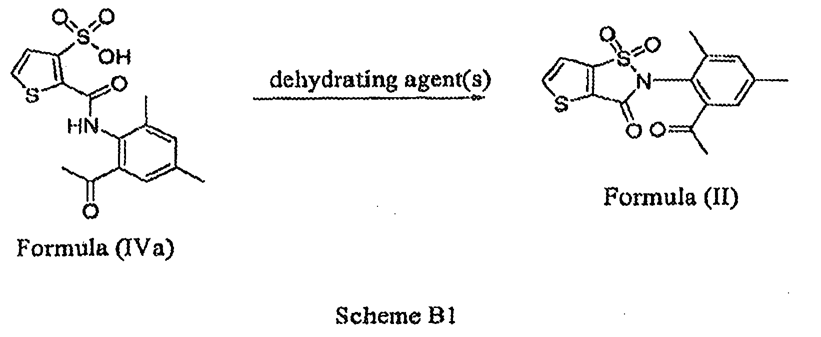

The preparation of the compound of Formula (IVa) may be prepared using methods known to those of ordinary skill in the art. For example, the compound of Formula (IVa) may be prepared using methods similar to those used in the preparation of the compound of Formula (IV).

The preparation of the compound of Formula (II) or a pharmaceutically acceptable salt, enol ether, enol ester, ketal, hemiketal, solvate or hydrate thereof as depicted in Scheme B or Scheme B1 may occur with any dehydrating agent or any combination of dehydrating agents according to a person of ordinary skill in the art. In some embodiments, the dehydrating agent is, or the combination of dehydrating agents are, generated in situ. In some embodiments, the dehydrating agent is (or the combination of dehydrating agents contains) thionyl chloride, sulfuryl chloride, 4-dimethylaminopyridine, a carbodiimide, an anhydride or a mixed anhydride, a phenol, or a compound of Formula (A): 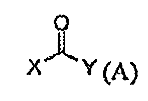 wherein each of X and Y is independently an unsubstituted or substituted heteroaryl group. In some embodiments, the dehydrating agent is (or combination of dehydrating agents contains) benzotriazole-1-yl-oxy-tris-(dimethylamino)-phosphonium hexafluorophosphate (BOP), N,N'-carbonyldiimidazole (CDI), 3-(diethoxyphosphoryloxy)-1,2,3-benzotriazin-4(3H)-one (DEPBT), 1-ethyl-3-(3-dimethyllaminopropyl)carbodiimide (EDC), 2-(7-aza-1H-benzotriazole-1-yl)-1,1,3,3-tetramethyluronium hexafluorophosphate (HATU), 2-(1H-benzotriazole-1-yl)-1,1,3,3-tetramethyluronium hexafluorophosphate (HBTU), 1-hydroxybenzotriazole (HOBt), benzotriazole-1-yl-oxy-tris-pyrrolidino-phosphonium hexafluorophosphate (PyBOP), 2-(1H-benzotriiazole-1-yl)-1,1,3,3-tetramethyluronium tetrafluoroborate (TBTU), O-(3,4-dihydro-4-oxo-1,2,3-benzotriazine-3-yl)-N,N,N,N-tetramethyluranium tetrafluoroborate (TDBTU), 3-(diethyloxyphosphoryloxy)-1,2,3-benzotriazin-4(3H)-one (DEPBT), dicyclohexylcarbodiimide (DCC), N,N'-diisopropylcarbodiimide (DIC), or 1-hydroxy-7-azabenzotriazole (HOAt). In some embodiments, the dehydrating agent is N,N'-carbonyldiimidazole.

The preparation of the compound of Formula (II) or a pharmaceutically acceptable salt, enol ether, enol ester, ketal, hemiketal, solvate or hydrate thereof as depicted in Scheme B or Scheme B1 may occur in any solvent or any combination of solvents. In some embodiments, the solvent is, or the combination of solvents contains, hexane, benzene, toluene, diethyl ether, chloroform, ethyl acetate, dichloromethane, carbon tetrachloride, 1,4-dioxane, tetrahydrofuran, glyme, diglyme, acetone, acetonitrile, dimethylformamide, dimethyl sulfoxide, dimethylacetamide, or N-methyl-2-pyrrolidone. In some embodiments, the solvent is ethyl acetate.

In some embodiments of the preparation of the compound of Formula (II) or a pharmaceutically acceptable salt, enol ether, enol ester, ketal, hemiketal, solvate or hydrate thereof as depicted in Scheme B or Scheme B1, the dehydrating agent is N,N'-carbonyldimidazole and the solvent is ethyl acetate.

The preparation of the compound of formula (II) or a pharmaceutically acceptable salt, enol ether, enol ester, ketal, hemiketal, solvate or hydrate thereof as depicted in Scheme B or Scheme B1 may occur at any reaction temperature according to a person of ordinary skill in the art. In some embodiments, the reaction temperature is from about 0°C to about 100°C. In some embodiments, the reaction temperature is from about 20°C to about 80°C. In some embodiments, the reaction temperature is from about 40° to about 60°C. In some embodiments, the reaction temperature is from about 20°C to about 30°C.

In some embodiments of the preparation of the compound of Formula (II) or a pharmaceutically acceptable salt, enol ether, enol ester, ketal, hemiketal, solvate or hydrate thereof as depicted in Scheme B or Scheme B1, the dehydrating agent is N,N'-carbonyldiimidazole, the solvent is ethyl acetate, and the reaction temperature is from about 20°C to about 30°C.

The preparation of the compound of Formula (II) or a pharmaceutically acceptable salt, enol ether, enol ester, ketal, hemiketal, solvate or hydrate thereof as depicted in Scheme B or Scheme B1 may occur at any reaction time according to a person of ordinary skill in the art. In some embodiments, the reaction time is from about 30 minutes to about 5 hours. In some embodiments, the reaction time is from about 3 hours to about 5 hours. In some embodiments, the reaction time is about 4 hours.

In some embodiments of die preparation of the compound of Formula (II) or a pharmaceutically acceptable salt, enol ether, enol ester, ketal, hemiketal, solvate or hydrate thereof as depicted in Scheme B or Scheme B1, the dehydrating agent is N,N'-carbonyldiimidazole, the solvent is ethyl acetate, the reaction temperature is from about 20°C to about 30°C, and the reaction time is about 4 hours.

The preparation of the compound of Formula (II) or a pharmaceutically acceptable salt, enol ether, enol ester, ketal, hemiketal, solvate or hydrate thereof as depicted in Scheme B or Scheme B1 may occur at any molar ratio according to a person of ordinary skill in the art. In some embodiments, the molar ratio of N,N'-carbonyldiimidazole to the compound of Formula (IV) is from about 5:1 to about 1:1. In some embodiments, the molar ratio of N,N'-carbonyldiimidazole to the compound of Formula (IV) is about 1.2:1.

In one embodiment of the preparation of the compound of Formula (II) or a pharmaceutically acceptable salt, enol ether, enol ester, ketal, hemiketal, solvate or hydrate thereof as depicted in Scheme B or Scheme B 1, the dehydrating agent is N,N'-carbonyldiimidazole, the solvent is ethyl acetate, the reaction temperature is from about 20°C to about 30°C, the reaction time is about 4 hours, and the molar ratio of N,N'-carbonyldiimidazole to the compound of Formula (IV) is about 1.2:1.

In another embodiment, provided is a process for preparing a compound of Formula (IV) or a pharmaceutically acceptable salt, enol ether, enol ester, ketal, hemiketal, solvate or hydrate thereof from a compound of Formula (VI) as depicted in Scheme C.

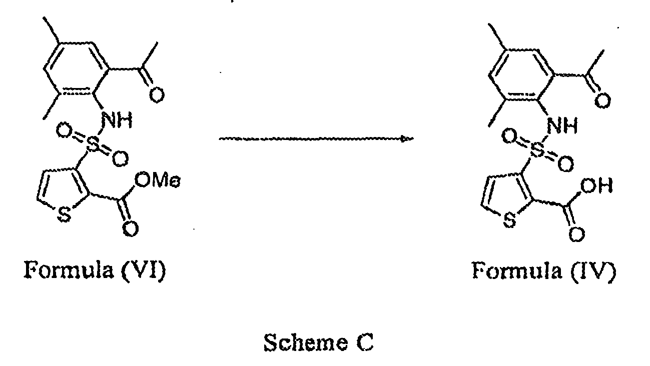

The preparation of the compound of Formula (IV) or a pharmaceutically acceptable salt, enol ether, enol ester, ketal, hemiketal, solvate or hydrate thereof as depicted in Scheme C may occur in the presence of any base or any combination of bases. In some embodiments, the base is (or the combination of bases are) generated in situ. In some embodiments, the base is (or the combination of bases contains) a hydroxide. In some embodiments, the base is (or the combination of bases contains) potassium hydroxide, sodium hydroxide, lithium hydroxide, magnesium hydroxide, calcium hydroxide, or barium hydroxide. In some embodiments, the base is sodium hydroxide.

The preparation of the compound of Formula (IV) or a pharmaceutically acceptable salt, enol ether, enol ester, ketal, hemiketal, solvate or hydrate thereof as depicted in Scheme C may occur in any solvent or any combination of solvents according to a person of ordinary skill in the art. In some embodiments, the solvent is (or the combination of solvents contains) hexane, benzene, toluene, diethyl ether, chloroform, ethyl acetate, dichloromethane, carbon tetrachloride, 1,4-dioxane, tetrahydrofuran, glyme, diglyme, acetone, acetonitrile, dimethylformamide, dimethyl sulfoxide, dimethylacetamide, or N-methyl-2-pyrrolidone. In some embodiments, the solvent is tetrahydrofuran.

In some embodiments of the preparation of the compound of Formula (IV) or a pharmaceutically acceptable salt, enol ether, enol ester, ketal, hemiketal, solvate or hydrate thereof as depicted in Scheme C, the base is sodium hydroxide and the solvent is tetrahydrofuran.

The preparation of the compound of Formula (IV) or a pharmaceutically acceptable salt, enol ether, enol ester, ketal, hemiketal, solvate or hydrate thereof as depicted in Scheme C may occur at any reaction temperature according to a person of ordinary skill in the art. In some embodiments, the reaction temperature is from about 0°C to about 100°C. In some embodiments, the reaction temperature is from about 20°C to about 80°C. In some embodiments, the reaction temperature is from about 40° to about 60°C. In some embodiments, the reaction temperature is from about 20°C to about 30°C.

In some embodiments of the preparation of the compound of Formula (IV) or a pharmaceutically acceptable salt, enol ether, enol ester, ketal, hemiketal, solvate or hydrate thereof as depicted in Scheme C, the base is sodium hydroxide, the solvent is tetrahydrofuran, and the reaction temperature is from about 20°C to about 30°C

The preparation of the compound of Formula (IV) or a pharmaceutically acceptable salt, enol ether, enol ester, ketal, hemiketal, solvate or hydrate thereof as depicted in Scheme C may occur at any reaction time according to a person of ordinary skill in the art. In some embodiments, the reaction time is from about 2 hours to about 36 hours.

In one embodiment of the preparation of the compound of Formula (IV) or a pharmaceutically acceptable salt, enol ether, enol ester, ketal, hemiketal, solvate or hydrate thereof as depicted in Scheme C, the base is sodium hydroxide, the solvent is tetrahydrofuran, the reaction temperature is from about 20°C to about 30°C, and the reaction time is from about 2 hours to about 36 hours.

The preparation of the compound of Formula (IV) or a pharmaceutically acceptable salt, enol ether, enol ester, ketal, hemiketal, solvate or hydrate thereof as depicted in Scheme C may occur in the presence of any acid or any combination of acids according to a person of ordinary skill in the art. In some embodiments, the acid is (or combination of acids are) generated in situ.

In another embodiment, provided is a process for preparing a compound of Formula (VI) or a pharmaceutically acceptable salt, enol ether, enol ester, ketal, orthoester, hemiketal, solvate or hydrate by reacting a compound of Formula (VII) with a compound of Formula (VIII) or a salt thereof as depicted in Scheme D.

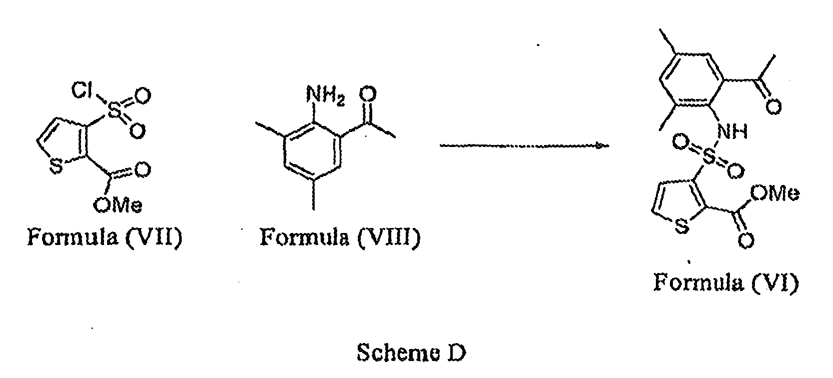

The preparation of the compound of Formula (VI) or a pharmaceutically acceptable salt, enol ether, enol ester, ketal, orthoester, hemiketal, solvate or hydrate thereof as depicted in Scheme D may occur in the presence of any base or any combination of bases according to a person of ordinary skill in the art. In some embodiments, the base is (or combination of bases are) generated in situ. In some embodiments, the base is (or combination of bases contains) a tertiary amine, aromatic amine, non-nucleophilic alkali metal amide, alkyl lithium, alkenyl lithium, alkynyl lithium, alk(en)(yn)yl lithium, alkyl magnesium halide, alkenyl magnesium halide, alkynyl magnesium halide, or alk(en)(yn)yl magnesium halide. In some embodiments, the base is (or combination of bases contains) dimethylethyl amine, trimethylamine, triethylamine, tributylamine, N-ethyldiisopropylamine, N-methylmorplioline, 1,8-diazabicyclo[5.4.0]-7-undecene, 1,5-diazabicyolo[4.3.0]-5-nonene; tetramethylenediamine, pyridine, collidine, lutidine, picoline, quinoline, or N,N-dimethylaniline. In some embodiments, the base is pyridine.

The preparation of the compound of Formula (VI) or a pharmaceutically acceptable salt, enol ether, enol ester, ketal, orthoester, hemiketal, solvate or hydrate thereof as depicted in Scheme D may occur at any reaction temperature according to a person of ordinary skill in the art. In some embodiments, the reaction temperature is from about 0°C to about 100°C. In some embodiments, the reaction temperature is from about 20°C to about 80°C. In some embodiments, the reaction temperature is from about 40° to about 60°C. In some embodiments, the reaction temperature is from about 20°C to about 30°C.

In some embodiments of th preparation of the compound of Formula (VI) or a pharmaceutically acceptable salt, enol ether, enol ester, ketal, orthoester, hemiketal, solvate or hydrate thereof as depicted in Scheme D, the base is pyridine and the reaction temperature is from about 20°C to about 30°C.

The preparation of the compound of Formula (VI) or a pharmaceutically acceptable salt, enol ether, enol ester, ketal, orthoester, hemiketal, solvate or hydrate thereof as depicted in Scheme D may occur at any reaction time according to a person of ordinary skill in the art. In some embodiments, the reaction time is from about 2 hours to about 36 hours.

In some embodiments of the preparation of the compound of Formula (VI) or a pharmaceutically acceptable salt, enol ether, enol ester, ketal, orthoester, hemiketal, solvate or hydrate thereof as depicted in Scheme D, the base is pyridine, the reaction temperature is from about 20°C to about 30°C, and the reaction time if from about 2 hours to about 36 hours.

The preparation of the compound of Formula (VI) or a pharmaceutically acceptable salt, enol ether, enol ester, ketal, orthoester hemiketal, solvate or hydrate thereof as depicted in Scheme D may occur in any solvent or any combination of solvents according to a person of ordinary skill in the art. Further, the preparation of the compound of Formula (VI) or a pharmaceutically acceptable derivative as depicted in Scheme D in the presence of a base or a combination of bases may occur in any solvent or combination of solvents according to a person of ordinary skill in the art. In some embodiments, the solvent is (or combination of solvents contains) hexane, benzene, toluene, diethyl ether, chloroform, ethyl acetate, dichloromethane, carbon tetrachloride, 1,4-dioxane, tetrahydrofuran, glyme, diglyme, acetone, acetonitrile, dimethylformamide, dimethyl sulfoxide, dimethylacetamide, and N-methyl-2-pyrrolidone.

The preparation of the compound of formula (VI) or a pharmaceutically acceptable salt, enol ether, enol ester, ketal, orthoester, hemiketal solvate or hydrate thereof as depicted in Scheme D may occur at any molar ratio according to a person of ordinary skill in the art. In some embodiments, the molar ratio of the compound of Formula (VIII) to the compound of Formula (VII) is from about 3:1 to about 1:1. In other embodiments, the molar ratio of the compound of Formula (VII) to the compound of Formula (VIII) is from about 3:1 to about 1:1. In some embodiments, the molar ratio of the compound of Formula (VIII) to the compound of Formula (VII) is about 1.1:1.

In one embodiment of the preparation of the compound of Formula (VI) or a pharmaceutically acceptable salt, enol ether, enol ester, ketal, orthoester, hemiketal, solvate or hydrate thereof as depicted in Scheme D, the base is pyridine, the reaction temperature is from about 20°C to about 30°C, the reaction time if from about 2 hours to about 36 hours, and the molar ratio of the compound of Formula (VIII) to the compound of Formula (VII) is about 1.1:1.

### EXAMPLES

### Example 1: Preparation of N-(2-acetyl-4,6-dimethylphenyl)-3-{[(3,4 dimethyl-5-isoxazolyl)amino]sulfonyl}-2-thiophenecarboxamide.

At 0° C; To 1.6 g 60% NaH in mineral oil was added a solution of 1.12 g of the compound of Formula (III) in 10 ml DMF. After stirring for 15 minutes a solution of 3.35 g of the compound of Formula (II) in 10 ml DMF was added drop wise. Alternatively, the compound of Formula (II) was added to the NaH first, followed by addition of the compound of Formule (III). As another alternative, the compounds of Formulae (II) and (III) were added simultaneously to the NaH. The reaction was stirred for 2 hours followed by a slow addition of 50 ml 2 N HCl (caution, excess NaH). The resulting suspension was extracted with toluene (4 x 25 ml). The organic layers were combined and washed with 2 N HCl (4 x 25 ml) followed by extraction with sat. Na-CO₃ (4 x 10 ml). The bicarbonate layers were combined and acidified with conc. HCl to pH ∼1-2 and extracted with EtOAc (3 x 25 ml). The EtOAc layers were combined and washed with 2 N HCl (25 ml), 2 N HCl/brine (25 ml), dried over MgSO₄ and concentrated *in vacuo* to yield 4.0 g TBC3711 as a tan solid with a purity >97%. Crystallization of 3.2 g crude N-(2-acetyl-4,6-dimethylphenyl)-3-{[(3,4 dimethyl-5-isoxazolyl)amino]sulfonyl}-2-thiophenecarboxamide from hot EtOH gave 2.75 g of the title compound as an off white solid in >99% purity by HPLC. ¹HNMR (500 MHz, DMSO-d₆): δ 1.65 (s, 3H), 2.08 (s, 3H), 2.22 (s, 3H), 2.32 (s, 3H), 2.47 (s, 3H), 7.27 (brs, 1H), 7.33 (d, J = 5.2 Hz, 1H), 7.38 (brs, I H), 7.86 (d, J = 5.2 Hz, 1H) and 10.26 (brs, 1H). ¹³C NMR (125 MHz, DMSO-d₆): δ 5.8, 10.3, 17.7, 20.4, 29.1, 105.7, 126.5, 127.9, 128.7, 129.8,133.9, 135.8, 136.2, 136,5, 138.3, 139.9, 155.2, 159.1, 161.4 and 200.5 ppm. MS (EaI) *m*/*z:* 446.08 [M-H]⁻.

### Example 2: Preparation of the Compound of Formula (II)

To a solution of 5.7 g of the compound of Formula (IV) in 60 ml EtOAc was added 3.1 g CDI. After stirring for 4 hours the reaction mixture was concentrated *in vacuo.* Crystallization from hot EtOAc gave 4.25 g of the compound of Formula (II) as a white solid in >99% by HPLC. ¹H NMR (400 MHz, CDCl₃): δ 2.4 (s, 3H), 2.43 (s, 3H), 2.53 (s, 3H), 7.35 (brs, 1H), 7.41 (d, J = 5.1 Hz, 1H), 7.45 (brs, 1H) and 7.91 (d, J = 5.1 Hz, 1H) ppm. MS (ESI) *m*/*z*: 693.08 [2M+H]⁺, and 336.00 [M+H]⁺.

### Example 3: Preparation of the Compound of Formula (IV)

To a solution of 6.25 g of the compound of Formula (VI) in 60 ml THF was added 30 ml 2N NaOH. After overnight stirring the reaction mixture was quenched with 10 ml conc. HCl followed by extraction with EtOAc (2 x). The organic layers were combined and washed with 2 N HCl (2 x), 2N HCl/brine (1 x), dried over MgSO₄ and concentrated *in vacuo* to yield 6.0 g of the compound of Formula (IV) as a light green sticky foam. Tituration with DCM gave the product as a dry white solid in >99% purity by HPLC. ¹H NMR (400 MHz, CDCl₃): δ 2.14 (s, 3H), 2.28 (s, 3H), 2.31 (s, 3H), 7.17 (d, J = 5.1 Hz, 1H), 7.27 (brs, 1H), 7.30 (brs, 1H), 7.64 (d, J = 5.1 Hz, 1H) and 8.91 (brs, 1H) ppm. MS (ESI) *m*/*z:* 720.08 [2M+H]⁺, 376.04 [M+Na]⁺and 354.06 [M+H]⁺.

### Example 4: Preparation of the Compound of Formula (VI)

To a solution of 4.4 g of the compound of Formula (VIII) in 20 ml pyridine was added 6.0 g the compound of Formula (VII). The reaction mixture was stirred overnight followed by addition of 50 ml 6 N HCl. The resulting suspension was extracted with DCM (2 x). The organic layers were combined and washed with 2 N HCl (2 x), 2 N HCl/brine, dried over MgSO₄ and concentrated *in vacuo.* Crystallization from hot EtOAc/hexanes (1 :1,20 ml) gave 6.33 g of the compound of Formula (VI) as an off-white solid in >99% purity by HPLC. ¹H NMR (400 MHz, CDCl₃): δ 2.14 (s, 3H), 2.32 (s, 3H), 2.42 (s, 3H), 4.04 (s, 3H), 7.18 (brs, 1H), 7.22 (brs, 1H), 7.28 (d, J = 5.1 Hz, 1H), 7.40 (d, J = 5.1 Hz, 1H) and 9.16 (brs, 1H) ppm. MS (ESI) *m*/*z*: 757.12 [2M+H]⁺ and 368.04 [M+H]⁺.
Since modifications would be apparent to those of skill in the art, this disclosure is intended to be limited only by the appended claims.

## Claims

1. A process for preparing a compound of Formula (I): 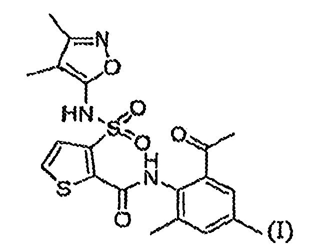 or a pharmaceutically acceptable salt, enol ether, enol ester, ketal, hemiketal, solvate or hydrate thereof, comprising a step of reacting a compound of Formule (II): 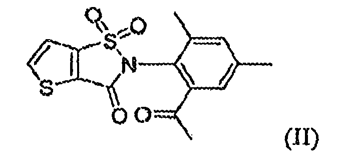 with a compound of Formule (III): 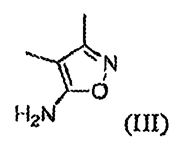 or a salt thereof.

2. The process of claim 1, wherein the reaction occurs in presence of a base or a combination of bases.

3. The process of claim 2, wherein the base is, or the combination of bases comprises, an alkali metal hydride.

4. The process of any of claims 2-3, wherein the base is, or the combination of bases comprises, sodium hydride, lithium hydride, or potassium hydride.

5. The process of any of claims 1-4, wherein the reaction occurs in a solvent or a combination of solvents, selected from:
1,4-dioxane, tetrahydrofuran, acetone, acetonitrile, dimethylformamide, dimethyl sulfoxide, glyme, diglyme, dimethylacetamide, or N-methyl-2-pyrolidone.

6. The process of claim 2, wherein the base is, or the combination of bases comprises, an alkyl lithium, an alkenyl lithium, an alkynyl lithium, an alk(en)(yn)yl lithium, an alkyl magnesium halide, an alkenyl magnesium halide, an alkynyl magnesium halide, an alk(en)(yn)yl magnesium halide, an alkali metal amide, tertiary amine, or aromatic amine.

7. The process of claim 6, wherein the reaction occurs in a solvent or a combination of solvents, selected from:
1,4-dioxane, tetrahydrofuran, dimethylformamide, dimethyl sulfoxide, glyme, diglyme, dimethylacetamide, or N-methyl-2-pyrrolidone.

8. The process of any of claims 1-7, wherein the reaction temperature is from about -78°C to about 100°C.

9. The process of any of claims 1-8, wherein the reaction time is from about 30 minutes to about 24 hours.

10. The process of any of claims 1-9, wherein the molar ratio of the compound of Formula (III) to the compound of Formula (II) is from about 3:1 to about 1:3.

11. The process of any of claims 1-10, wherein the molar ratio of the compound of Formula (III) to the compound of Formula (II) is about 1:1.

12. The process of any of claims 1-11, further comprising preparing the compound of Formula (II) by reacting a compound of Formula (IV): 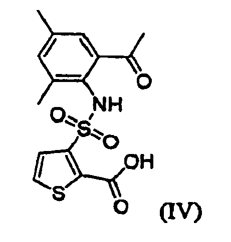 with a dehydrating agent or a combination of dehydrating agents.

13. The process according to claim 12, wherein the dehydrating agent is, or the combination of dehydrating agents comprises, thionyl chloride, sulfury chloride, a carbodiimide, an anhydride or a mixed anhydride, a phenol, or a compound of Formula (A): 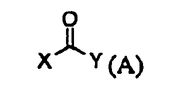 wherein each of X and Y is independently an unsubstituted or substituted heteroaryl group.

14. The process of any of claims 12-13, wherein the dehydrating agent is, or the combination of dehydrating agents comprises, benzotriazola-1-yl-oxy-tris-(dimethylamino)-phosphonium hexafluorophosphate, N,N'-carbonyldiimidazole, 3-(diethoxyphosphoryloxy)-1,2,3-benzotriazin-4(3H)-one, 1-methyl-3-(3-dimethyllaminopropyl)carbodiimide, 2-(7-aha- 1H-benzotriazole-1-yl)-1, 1,3,3-tetramethyluronium hexafluorophosphate, 2-(1H-benzotriazote-1-yl)-1,1,3,3-tetramethyluronium hexafluorophosphate, 1-hydroxybenzotriazole, benzotriazole-1-yl-oxy-tris-pyrrolidino-phosphonium hexafluorophosphate, 2-(1H-benzotriazote-1-yl)-1,1,3,3-tetramethyluronium tetrafluoroborate, O-(3,4-dihydro-4-oxo-1,2,3-benzotriazine-3-yl)-N,N,N,N-tetramethyluronium tetrafluoroborate, 3-(diethyloxyphosphoryloxy)-1,2,3-benzotriazin-4(3*H*)-one, dicyclohexylcarbodiimide, N,N'-diisopropylcarbodiimide, or 1-hydroxy-7-azabenzotriazole, nitrophenol, pentafluorophenol, or phenol.

15. The process of any of claims 12 to 14, wherein the reaction occurs in a solvent or a combination of solvents selected from :
hexane, benzene, toluene, diethyl ether, chloroform, ethyl acetate, dichloromethane, carbon tetrachloride, 1,4-dioxane, tetrahydrofuran, glyme, diglyme, acetone, acetonitrile, dimethylformamide, dimethyl sulfoxide, dimethylacetamide, or N-methyl-2-pyrrolidone.

16. The process of any of claims 12 to 15, wherein the reaction temperature is from about 0°C to about 100°C.

17. The process of any of claims 12 to 16, wherein the reaction time is from about 30 minutes to about 24 hours.

18. The process of any of claims 12 to 17, wherein the molar ratio of N,N'-carbonyldiimidazole to the compound of Formula (IV) is from about 5:1 to about 1:1.

19. The process of any of claims 12 to 18, further comprising preparing the compound of Formula (IV) by reacting a compound of Formula (VI): 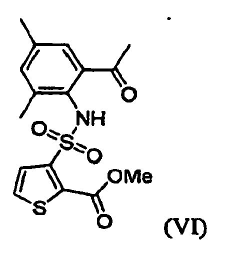 with a base or a combination of bases or alternatively with an acid or a combination of acids.

20. The process according to claim 19, wherein the base is, or combination of bases comprises, a hydroxide.

21. The process of any of claims 19 to 20, wherein the base is, or combination of bases comprises, potassium hydroxide, sodium hydroxide, lithium hydroxide, magnesium hydroxide, calcium hydroxide, or barium hydroxide.

22. The process of any of claims 19 to 21, wherein the reaction occurs in a solvent or a combination of solvents selected from :
hexane, benzene, toluene, diethyl ether, chloroform, ethyl acetate, dichloromethane, carbon tetrachloride, 1,4-dioxane, tetrahydrofuran, glyme, diglyme, acetone, acetonitrile, dimethylformamide, dimethyl sulfoxide, dimethylacetamide, or N-methyl-2-pyrrolidone.

23. The process of any of claims 19 to 22, wherein the reaction temperature is from about 0°C to about 100°C.

24. The process of any of claims 19 to 23, wherein the reaction time is from about 2 hours to about 36 hours.

25. The process of any of claims 19 to 24, further comprising preparing the compound of Formula (VI) by reacting a compound of Formula (VII): 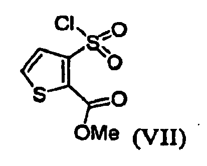 with a compound of Formula (VIII):

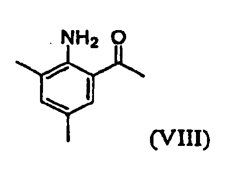

26. The process according to claim 25, wherein the reaction occurs in the presence of a base or a combination of bases selected from:
a tertiary amine, aromatic amine, non-nucleophilic alkali metal amide, alkyl lithium, alkenyl lithium, alkynyl lithium, alk(en)(yn)yl lithium, alkyl magnesium halide, alkenyl magnesium halide, alkynyl magnesium halide, or alk(en)(yn)yl magnesium halide.

27. The process according to claim 26, wherein the base is, or the combination of bases comprises, dimethylethyl amine, trimethylamine, triethylamine, tributylamine, N-ethyldiisopropylamine, N-methylmorpholine, 1,8-diazabicyclo[5.4.0]-7-undecene, 1,5-diazabicyclo[4.3.0]-5-nonene, tetramethylenediamine, pyridine, picoline, quinoline, or N,N-dimethylaniline.

28. The process of any of claims 25 to 27, wherein the reaction occurs in a solvent or a combination of solvents selected from :
hexane, benzene, toluene, diethyl ether, chloroform, ethyl acetate, dichloromethane, carbon tetrachloride, 1,4-dioxane, tetrahydrofuran, glyme, diglyme, acetone, acetonitrile, dimethylformamide, dimethyl sulfoxide, dimethylacetamide, and N-memyl-2-pyrrolidone.

29. The process of any of claims 25 to 28, wherein the reaction temperature is from about 0°C to about 100°C.

30. The process of any of claims 25 to 29, wherein the reaction time is from about 2 hours to about 36 hours.

31. The process of any of claims 25 to 30, wherein the molar ratio of the compound of Formula (VIII) to the compound of Formula (VII) is from about 3:1 to about 1:3.

32. A compound of Formula (II): 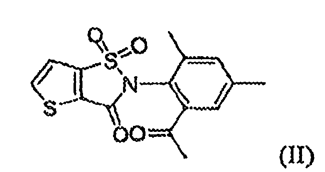 or a pharmaceutically acceptable salt, enol ether, enol ester, ketal, hemiketal, solvate or hydrate thereof.

33. A compound of Formula (IV): 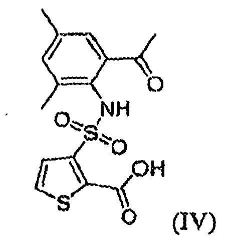 or a pharmaceutically acceptably salt, enol ether, enol ester, ketal, hemiketal, solvate or hydrate thereof.

34. A compound of Formula (VI): 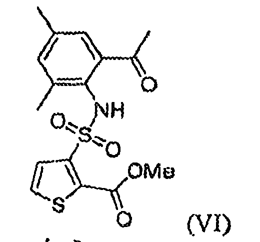 or a pharmaceutically acceptable salt, enol ether, enol ester, ketal, orthoester, hemiketal, solvate or hydrate thereof.

## Patentansprüche

1. Verfahren zur Herstellung einer Verbindung der Formel (I) : 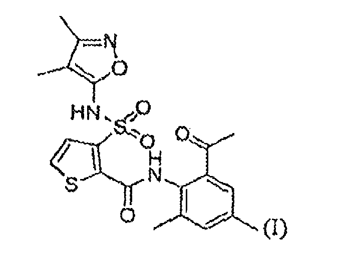 oder eines pharmazeutisch verträglichen Salzes, Enolethers, Enolesters, Ketals, Hemiketals, Solvats oder Hydrats davon, umfassend einen Schritt des Umsetzens einer Verbindung der Formel (II): 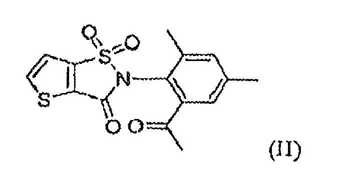 mit einer Verbindung der Formel (III): 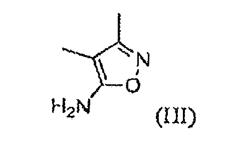 oder eines Salzes davon.

2. Verfahren gemäß Anspruch 1, wobei die Umsetzung in Gegenwart einer Base oder einer Kombination von Basen stattfindet.

3. Verfahren gemäß Anspruch 2, wobei die Base ein Alkalimetallhydrid ist oder die Kombination von Basen ein Alkalimetallhydrid umfasst.

4. Verfahren gemäß einem beliebigen der Ansprüche 2 bis 3, wobei die Base Natriumhydrid, Lithiumhydrid oder Kaliumhydrid ist oder die Kombination von Basen Natriumhydrid, Lithiumhydrid oder Kaliumhydrid umfasst.

5. Verfahren gemäß einem beliebigen der Ansprüche 1 bis 4, wobei die Umsetzung in einem Lösungsmittel oder einer Kombination von Lösungsmitteln stattfindet, ausgewählt aus 1,4-Dioxan, Tetrahydrofuran, Aceton, Acetonitril, Dimethylformamid, Dimethylsulfoxid, Glyme, Diglyme, Dimethylacetamid oder N-Methyl-2-pyrrolidon.

6. Verfahren gemäß Anspruch 2, wobei die Base ist, oder die Kombination von Basen umfasst: ein Alkyllithium, ein Alkenyllithium, ein Alkinyllithium, ein Alk(en)(in)yllithium, ein Alkylmagnesiumhalogenid, ein Alkenylmagnesiumhalogenid, ein Alkinylmagnesiumhalogenid, ein Alk(en)(in)ylmagnesium-halogenid, ein Alkalimetallamid, ein tertiäres Amin oder ein aromatisches Amin.

7. Verfahren gemäß Anspruch 6, wobei die Umsetzung in einem Lösungsmittel oder einer Kombination von Lösungsmitteln erfolgt, ausgewählt aus 1,4-Dioxan, Tetrahydrofuran, Dimethylformamid, Dimethylsulfoxid, Glyme, Diglyme, Dimethylacetamid oder N-Methyl-2-pyrrolidon.

8. Verfahren gemäß einem beliebigen der Ansprüche 1 bis 7, wobei die Reaktionstemperatur zwischen etwa -78°C und etwa 100°C beträgt.

9. Verfahren gemäß einem beliebigen der Ansprüche 1 bis 8, wobei die Reaktionszeit von etwa 30 Minuten bis etwa 24 Stunden beträgt.

10. Verfahren gemäß einem beliebigen der Ansprüche 1 bis 9, wobei das molare Verhältnis der Verbindung der Formel (III) zur Verbindung der Formel (II) von etwa 3:1 bis etwa 1:3 beträgt.

11. Verfahren gemäß einem beliebigen der Ansprüche 1 bis 10, wobei das molare Verhältnis der Verbindung der Formel (III) zur Verbindung der Formel (II) etwa 1:1 beträgt.

12. Verfahren gemäß einem beliebigen der Ansprüche 1 bis 11, des weiteren umfassend Herstellen der Verbindung der Formel (II) durch Umsetzen einer Verbindung der Formel (IV): 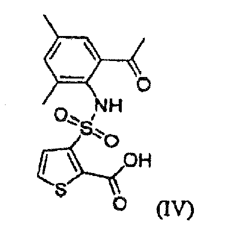 mit einem Entwässerungsmittel oder einer Kombination von Entwässerungsmitteln.

13. Verfahren gemäß Anspruch 12, wobei das Entwässerungsmittel oder die Kombination aus Entwässerungsmitteln Thionylchlorid, Sulfurylchlorid, ein Carbodiimid, ein Anhydrid oder ein gemischtes Anhydrid, ein Phenol oder eine Verbindung der Formel (A) umfasst: 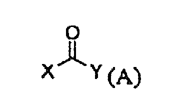 wobei jedes von X und Y unabhängig eine unsubstituierte oder substituierte Heteroarylgruppe darstellt.

14. Verfahren gemäß einem beliebigen der Ansprüche 12 bis 13, wobei das Entwässerungsmittel oder die Kombination von Entwässerungsmitteln Benzotriazol-1-yl-oxy-tris-(dimethylamino)-phosphoniumhexafluorphosphat, N,N'-Carbonyldiimidazol, 3-(Di-ethoxyphosphoryloxy)-1,2,3-benzotriazin-4(3H)-on, 1-Ethyl-3-(3-Dimethylaminopropyl)carbodiimid, 2-(7-Aza-1H-benzotriazol-1-yl)-1,1,3,3-tetramethyluroniumhexafluorphosphat, 2-(1H-Benzotriazol-1-yl)-1,1,3,3,-tetramethyluroniumhexafluorphosphat, 1-Hydroxybenzotriazol, Benzotriazol-1-yl-oxy-tris-pyrrolidino-phosphoniumhexafluorphosphat, 2-(1H-Benzotriazol-1-yl)-1,1,3,3-tetramethyluroniumtetrafluorborat, 0-(3,4-Dihydro-4-oxo-1,2,3-benzotriazin-3-yl)-N,N,N,N-tetramethyluroniumtetrafluorborat, 3-(Diethyloxyphosphoryloxy)-1,2,3-benzotriazin-4(3H)-on, Dicyclohexylcarbodiimid, N,N'-Diisopropylcarbodiimid oder 1-Hydroxy-7-azabenzotriazol, Nitrophenol, Pentafluorphenol oder Phenol umfasst.

15. Verfahren gemäß einem beliebigen der Ansprüche 12 bis 14, wobei die Umsetzung in einem Lösungsmittel oder einer Kombination von Lösungsmitteln geschieht, ausgewählt aus: Hexan, Benzol, Toluol, Diethylether, Chloroform, Ethylacetat, Dichlormethan, Kohlenstofftetrachlorid, 1,4-Dioxan, Tetrahydrofuran, Glyme, Diglyme, Aceton, Acetonitril, Dimethylformamid, Dimethylsulfoxid, Dimethylacetamid oder N-Methyl-2-pyrrolidon.

16. Verfahren gemäß einem beliebigen der Ansprüche 12 bis 15, wobei die Reaktionstemperatur von etwa 0°C bis etwa 100°C beträgt.

17. Verfahren gemäß einem beliebigen der Ansprüche 12 bis 16, wobei die Reaktionszeit von etwa 30 Minuten bis etwa 24 Stunden beträgt.

18. Verfahren gemäß einem beliebigen der Ansprüche 12 bis 17, wobei das molare Verhältnis von N,N'-Carbonyldiimidazol zur Verbindung der Formel (IV) von etwa 5:1 bis etwa 1:1 beträgt.

19. Verfahren gemäß einem beliebigen der Ansprüche 12 bis 18, des weiteren umfassend Herstellen der Verbindung der Formel (IV) durch Umsetzen einer Verbindung der Formel (VI): 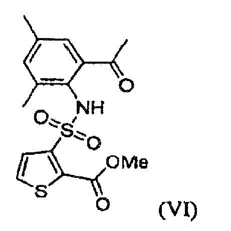 mit einer Base oder einer Kombination von Basen oder alternativ mit einer Säure oder einer Kombination von Säuren.

20. Verfahren gemäß Anspruch 19, wobei die Base ein Hydroxid ist, oder die Kombination von Basen ein Hydroxid umfasst.

21. Verfahren gemäß einem beliebigen der Ansprüche 19 bis 20, wobei die Base ist oder die Kombination von Basen umfasst: Kaliumhydroxid, Natriumhydroxid, Lithiumhydroxid, Magnesiumhydroxid, Calciumhydroxid oder Bariumhydroxid.

22. Verfahren gemäß einem beliebigen der Ansprüche 19 bis 21, wobei die Umsetzung in einem Lösungsmittel oder einer Kombination von Lösungsmitteln erfolgt, ausgewählt aus: Hexan, Benzol, Toluol, Diethylether, Chloroform, Ethylacetat, Dichlormethan, Kohlenstofftetrachlorid, 1,4-Dioxan, Tetrahydrofuran, Glyme, Diglyme, Aceton, Acetonitril, Dimethylformamid, Dimethylsulfoxid, Dimethylacetamid oder N-Methyl-2-pyrrolidon.

23. Verfahren gemäß einem beliebigen der Ansprüche 19 bis 22, wobei die Reaktionstemperatur von etwa 0°C bis etwa 100°C beträgt.

24. Verfahren gemäß einem beliebigen der Ansprüche 19 bis 23, wobei die Umsetzungszeit von etwa 2 Stunden bis etwa 36 Stunden beträgt.

25. Verfahren gemäß einem beliebigen der Ansprüche 19 bis 24, des weiteren umfassend Herstellen der Verbindung der Formel (VI) durch Umsetzen einer Verbindung der Formel (VII): 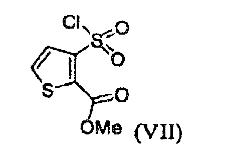 mit einer Verbindung der Formel (VIII): 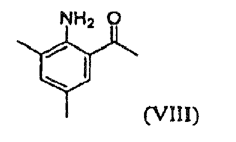 oder eines Salzes davon.

26. Verfahren gemäß Anspruch 25, wobei die Umsetzung in Gegenwart einer Base oder einer Kombination von Basen geschieht, ausgewählt aus: einem tertiärem Amin, aromatischen Amin, nicht-nukleophilem Alkalimetallamid, Alkyllithium, Alkenyllithium, Alkinyllithium, Alk(en)(in)yllithium, Alkylmagnesiumhalogenid, Alkenylmagnesiumhalogenid, ein Alkinylmagnesiumhalogenid oder Alk(en)(in)ylmagnesiumhalogenid.

27. Verfahren gemäß Anspruch 26, wobei die Base ist, oder die Kombination von Basen umfasst: Dimethylethylamin, Trimethylamin, Triethylamin, Tributylamin, N-Ethyldiisopropylamin, N-Methylmorpholin, 1,8-Diazabicyclo[5.4.0]-7-undecen, 1,5-Diazabicyclo[4.3.0]-5-nonen, Tetramethylendiamin, Pyridin, Picolin, Chinolin oder N,N-Dimehtylanilin.

28. Verfahren gemäß einem beliebigen der Ansprüche 25 bis 27, wobei die Umsetzung in einem Lösungsmittel oder einer Kombination von Lösungsmitteln erfolgt, ausgewählt aus: Hexan, Benzol, Toluol, Diethylether, Chloroform, Ethylacetat, Dichlormethan, Kohlenstofftetrachlorid, 1,4-Dioxan, Tetrahydrofuran, Glyme, Diglyme, Aceton, Acetonitril, Dimethylformamid, Dimethylsulfoxid, Dimethylacetamid oder N-Methyl-2-pyrrolidon.

29. Verfahren gemäß einem beliebigen der Ansprüche 25 bis 28, wobei die Reaktionstemperatur von etwa 0°C bis etwa 100°C beträgt.

30. Verfahren gemäß einem beliebigen der Ansprüche 25 bis 29, wobei die Umsetzungszeit von etwa 2 Stunden bis etwa 36 Stunden beträgt.

31. Verfahren gemäß einem beliebigen der Ansprüche 25 bis 30, wobei das molare Verhältnis der Verbindung der Formel (VIII) zur Verbindung der Formel (VII) von etwa 3:1 bis etwa 1:3 beträgt.

32. Verbindung der Formel (II): 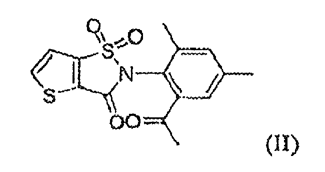 oder ein pharmazeutisch verträgliches(r) Salz, Enolether, Enolester, Ketal, Hemiketal, Solvat oder Hydrat davon.

33. Verbindung der Formel (IV): 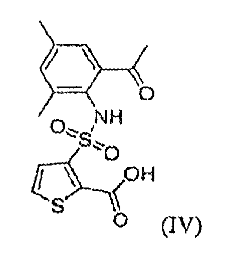 oder ein pharmazeutisch verträgliches(r) Salz, Enolether, Enolester, Ketal, Hemiketal, Solvat oder Hydrat davon.

34. Verbindung der Formel (VI): 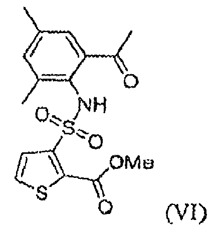 oder ein pharmazeutisch verträgliches(r) Salz, Enolether, Enolester, Ketal, Orthoester, Hemiketal, Solvat oder Hydrat davon.

## Revendications

1. Procédé pour la préparation d'un composé de Formule (I) : 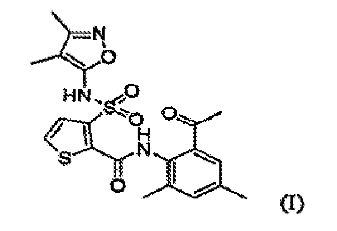 ou d'un sel, éther d'énol, ester d'énol, cétal, hémicétal, produit de solvatation ou hydrate pharmaceutiquement acceptable de celui-ci, comprenant une étape de réaction d'un composé de Formule (II) : 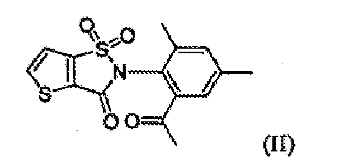 avec un composé de Formule (III) : 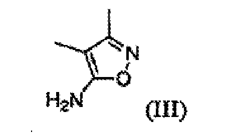 ou un sel de celui-ci.

2. Procédé selon la revendication 1, dans lequel la réaction a lieu en présence d'une base ou d'une association de bases.

3. Procédé selon la revendication 2, dans lequel la base est, ou l'association de bases comprend, un hydrure de métal alcalin.

4. Procédé selon l'une quelconque des revendications 2 et 3, dans lequel la base est, ou l'association de bases comprend, l'hydrure de sodium, l'hydrure de lithium ou l'hydrure de potassium.

5. Procédé selon l'une quelconque des revendications 1 à 4, dans lequel la réaction a lieu dans un solvant ou une association de solvants, choisi(s) parmi : le 1,4-dioxanne, le tétrahydrofuranne, l'acétone, l'acétonitrile, le diméthylformamide, le diméthylsulfoxyde, le glyme, le diglyme, le diméthylacétamide ou la N-méthyl-2-pyrrolidone.

6. Procédé selon la revendication 2, dans lequel la base est, ou l'association de bases comprend, un alkyl-lithium, un alcényl-lithium, un alcynyl-lithium, un alc(én)(yn)yl-lithium, un halogénure d'alkylmagnésium, un halogénure d'alcénylmagnésium, un halogénure d'alcynylmagnésium, un halogénure d'alc(én)(yn)ylmagnésium, un amidure de métal alcalin, une amine tertiaire ou une amine aromatique.

7. Procédé selon la revendication 6, dans lequel la réaction a lieu dans un solvant ou une association de solvants, choisi(s) parmi : le 1,4-dioxanne, le tétrahydrofuranne, le diméthylformamide, le diméthylsulfoxyde, le glyme, le diglyme, le diméthylacétamide ou la N-méthyl-2-pyrrolidone.

8. Procédé selon l'une quelconque des revendications 1 à 7, dans lequel la température de réaction est d'environ -78°C à environ 100°C.

9. Procédé selon l'une quelconque des revendications 1 à 8, dans lequel le temps de réaction est d'environ 30 minutes à environ 24 heures.

10. Procédé selon l'une quelconque des revendications 1 à 9, dans lequel le rapport molaire du composé de Formule (III) au composé de Formule (II) est d'environ 3:1 à environ 1:3.

11. Procédé selon l'une quelconque des revendications 1 à 10, dans lequel le rapport molaire du composé de Formule (III) au composé de Formule (II) est d'environ 1:1.

12. Procédé selon l'une quelconque des revendications 1 à 11, comprenant de plus la préparation du composé de Formule (II) par réaction d'un composé de Formule (IV) : 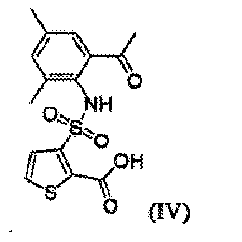 avec un agent déshydratant ou une association d'agents déshydratants.

13. Procédé selon la revendication 12, dans lequel l'agent déshydratant est, ou l'association d'agents déshydratants comprend, le chlorure de thionyle, le chlorure de sulfuryle, un carbodiimide, un anhydride ou un anhydride mixte, un phénol ou un composé de Formule (A) : 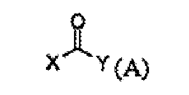 où chacun de X et Y est indépendamment un groupe hétéroaryle substitué ou non substitué.

14. Procédé selon l'une quelconque des revendications 12 et 13, dans lequel l'agent déshydratant est, ou l'association d'agents déshydratants comprend, l'hexafluorophosphate de benzotriazole-1-yl-oxy-tris(diméthylamino)phosphonium, le N,N'-carbonyldiimidazole, 1 a 3-(diéthoxyphosphoryloxy)-1,2,3-benzotriazine-4(3H)-one, 1 e 1-éthyl-3-(3-diméthylaminopropyl)carbodiimide, l'hexafluorophosphate d e 2-(7-aza-1H-benzotriazole-1-yl)-1,1,3,3-tétraméthyluronium, l'hexafluorophosphate de 2-(1H-benzotriazole-1-yl)-1,1,3,3-tétraméthyluronium, le 1-hydroxybenzotriazole, l'hexafluorophosphate de benzotriazole-1-yl-oxy-tris-pyrrolidinophosphonium, le tétrafluoroborate de 2-(1H-benzotriazole-1-yl)-1,1,3,3-tétraméthyluronium, le tétrafluoroborate de O-(3,4-dihydro-4-oxo-1,2,3-benzotriazine-3-yl)-N,N,N,N-tétraméthyluronium, la 3-(diéthyloxyphosphoryloxy) -1, 2, 3-benzo-triazine-4(3H)-one, le dicyclohexylcarbodiimide, le N,N'-diisopropylcarbodiimide ou le 1-hydroxy-7-azabenzotriazole, le nitrophénol, le pentafluorophénol ou le phénol.

15. Procédé selon l'une quelconque des revendications 12 à 14, dans lequel la réaction a lieu dans un solvant ou une association de solvants choisi(s) parmi : l'hexane, le benzène, le toluène, l'éther de diéthyle, le chloroforme, l'acétate d'éthyle, le dichlorométhane, le tétrachlorure de carbone, le 1,4-dioxanne, le tétrahydrofuranne, le glyme, le diglyme, l'acétone, l'acétonitrile, le diméthylformamide, le diméthylsulfoxyde, le diméthylacétamide ou la N-méthyl-2-pyrrolidone.

16. Procédé selon l'une quelconque des revendications 12 à 15, dans lequel la température de réaction est d'environ 0°C à environ 100°C.

17. Procédé selon l'une quelconque des revendications 12 à 16, dans lequel le temps de réaction est d'environ 30 minutes à environ 24 heures.

18. Procédé selon l'une quelconque des revendications 12 à 17, dans lequel le rapport molaire du N,N'-carbonyldiimidazole au composé de Formule (IV) est d'environ 5:1 à environ 1:1.

19. Procédé selon l'une quelconque des revendications 12 à 18, comprenant de plus la préparation du composé de Formule (IV) par réaction d'un composé de Formule (VI) : 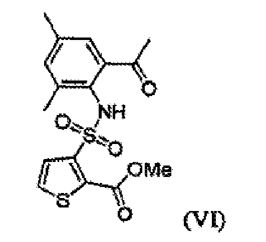 avec une base ou une association de bases ou, en variante, avec un acide ou une association d'acides.

20. Procédé selon la revendication 19, dans lequel la base est, ou l'association de bases comprend, un hydroxyde.

21. Procédé selon l'une quelconque des revendications 19 à 20, dans lequel la base est, ou l'association de bases comprend, l'hydroxyde de potassium, l'hydroxyde de sodium, l'hydroxyde de lithium, l'hydroxyde de magnésium, l'hydroxyde de calcium ou l'hydroxyde de baryum.

22. Procédé selon l'une quelconque des revendications 19 à 21, dans lequel la réaction a lieu dans un solvant ou une association de solvants, choisi(s) parmi : l'hexane, le benzène, le toluène, l'éther de diéthyle, le chloroforme, l'acétate d'éthyle, le dichlorométhane, le tétrachlorure de carbone, le 1,4-dioxanne, le tétrahydrofuranne, le glyme, le diglyme, l'acétone, l'acétonitrile, le diméthylformamide, le diméthylsulfoxyde, le diméthylacétamide ou la N-méthyl-2-pyrrolidone.

23. Procédé selon l'une quelconque des revendications 19 à 22, dans lequel la température de réaction est d'environ 0°C à environ 100°C.

24. Procédé selon l'une quelconque des revendications 19 à 23, dans lequel le temps de réaction est d'environ 2 heures à environ 36 heures.

25. Procédé selon l'une quelconque des revendications 19 à 24, comprenant de plus la préparation du composé de Formule (VI) par réaction d'un composé de Formule (VII) : 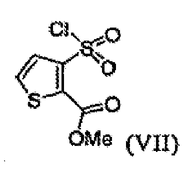 avec un composé de Formule (VIII) : 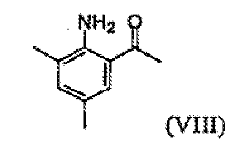 ou un sel de celui-ci.

26. Procédé selon la revendication 25, dans lequel la réaction a lieu en présence d'une base ou d'une association de bases choisie(s) parmi : une amine tertiaire, une amine aromatique, un amidure de métal alcalin non nucléophile, un alkyl-lithium, un alcényl-lithium, un alcynyl-lithium, un alc (én) (yn)yl-lithium, un halogénure d'alkylmagnésium, un halogénure d'alcénylmagnésium, un halogénure d' alcynylmagnésium ou un halogénure d'alc(én)(yn)ylmagnésium.

27. Procédé selon la revendication 26, dans lequel la base est, ou l'association de bases comprend, la diméthyléthylamine, la triméthylamine, la triéthylamine, la tributylamine, la N-éthyldiisopropylamine, la N-méthylmorpholine, le 1,8-diazabicyclo[5.4.0]-7-undécène, le 1,5-diazabicyclo[4.3.0]-5-nonène, la tétraméthylènediamine, la pyridine, la picoline, la quinoléine ou la N,N-diméthylaniline.

28. Procédé selon l'une quelconque des revendications 25 à 27, dans lequel la réaction a lieu dans un solvant ou une association de solvants choisi(s) parmi : l'hexane, le benzène, le toluène, l'éther de diéthyle, le chloroforme, l'acétate d'éthyle, le dichlorométhane, le tétrachlorure de carbone, le 1,4-dioxanne, le tétrahydrofuranne, le glyme, le diglyme, l'acétone, l'acétonitrile, le diméthylformamide, le diméthylsulfoxyde, le diméthylacétamide et la N-méthyl-2-pyrrolidone.

29. Procédé selon l'une quelconque des revendications 25 à 28, dans lequel la température de réaction est d'environ 0°C à environ 100°C.

30. P r o cédé selon l'une quelconque des revendications 25 à 29, dans lequel le temps de réaction est d'environ 2 heures à environ 36 heures.

31. Procédé selon l'une quelconque des revendications 25 à 30, dans lequel le rapport molaire du composé de Formule (VIII) au composé de Formule (VII) est d'environ 3:1 à environ 1:3.

32. Composé de Formule (II) : 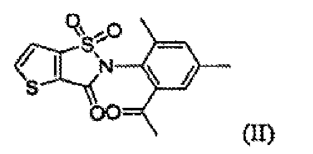 ou un sel, éther d'énol, ester d'énol, cétal, hémicétal, produit de solvatation ou hydrate pharmaceutiquement acceptable de celui-ci.

33. Composé de Formule (IV) : 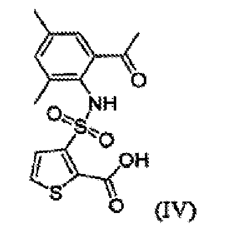 ou un sel, éther d'énol, ester d'énol, cétal, hémicétal, produit de solvatation ou hydrate pharmaceutiquement acceptable de celui-ci.

34. Composé de Formule (VI) : 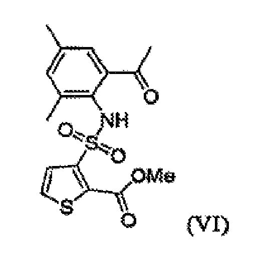 ou un sel, éther d'énol, ester d'énol, cétal, orthoester, hémicétal, produit de solvatation ou hydrate pharmaceutiquement acceptable de celui-ci.
